# EUROPEAN PATENT SPECIFICATION

(11) **EP 1 267 925 B1**
(45) Date of publication and mention of the grant of the patent: **13.06.2007**
(21) Application number: 01911971.8
(22) Date of filing: 20.03.2001
(51) Int. Cl.: A61K 39/395, A61K 38/12, A61K 31/70, A61P 31/10, C07K 16/14, A61K 47/38, G01N 33/569

(54) **TREATMENT OF FUNGAL INFECTION WITH POLYENE OR BETA GLUCAN SYNTHASE INHIBITOR ANTIFUNGALS COMBINED WITH ANTI HSP90 ANTIBODIES**
BEHANDLUNG VON PILZINFEKTION MIT POLYENEN ODER BETA GLUCAN SYNTHESE HEMMERN KOMBINIERT MIT ANTI-HSP ANTIKÖRPERN
TRAITEMENT DES INFECTIONS FONGIQUES AVEC DES ANTIFONGIQUES A BASE D'INHIBITEUR DE SYNTHASE POLYENE OU BETA GLUCANE COMBINES A DES ANTICORPS ANTI-HSP90

(30) Priority: 06.04.2000 GB 0008305
(43) Date of publication of application: 02.01.2003
(60) Divisional application: 07075188.8
(73) Proprietor: NEUTEC PHARMA PLC, Manchester M13 9WL (GB)
(72) Inventor: BURNIE, James Peter, Alderley Edge, Cheshire SK9 7PY (GB)
(74) Representative: Arends, William Gerrit
(86) International application number: PCT/GB2001/001195
(87) International publication number: WO 2001/076627

(56) References cited:
- EP-A- 0 861 892
- WO-A-94/04676
- R. MATTHEWS AND J. BURNIE: "The role of hsp 90 in fungal infection" IMMUNOLOGY TODAY, vol. 13, no. 9, 1992, pages 345-348, XP001008081
- MATTHEWS R C ET AL: "AUTOANTIBODY TO HEAT-SHOCK PROTEIN 90 CAN MEDIATE PROTECTION AGAINST SYSTEMIC CANDIDOSIS" IMMUNOLOGY,GB,OXFORD, vol. 74, no. 1, 1 September 1991 (1991-09-01), pages 20-24, XP002067298 ISSN: 0019-2805

### SEQUENCE LISTING

<110> NeuTec Pharma plc
<120> Treatment of fungal infections
<130> N00/0101/PCT
<140>
   <141>
<160> 2
<170> PatentIn Ver. 2.1
<210> 1
   <211> 9
   <212> PRT
   <213> Candida sp.
<400> 1
   Leu Lys Val Ile Arg Lys Asn Ile Val
    1               5
<210> 2
   <211> 248
   <212> PRT
   <213> Artificial Sequence
<220>
   <223> Description of Artificial Sequence:Synthetic
<400> 2
   His Met Ala Glu Val Gln Leu Val Glu Ser Gly Ala Glu Val Lys Lys
    1               5                  10                  15
   Pro Gly Glu Ser Leu Arg Ile Ser Cys Lys Gly Ser Gly Cys Ile Ile
               20                  25                  30
   Ser Ser Tyr Trp Ile Ser Trp Val Arg Gln Met Pro Gly Lys Gly Leu
           35                  40                  45
   Glu Trp Met Gly Lys Ile Asp Pro Gly Asp Ser Tyr Ile Asn Tyr Ser
       50                  55                  60
   Pro Ser Phe Gln Gly His Val Thr Ile Ser Ala Asp Lys Ser Ile Asn
   65                  70                  75                  80
   Thr Ala Tyr Leu Gln Trp Asn Ser Leu Lys Ala Ser Asp Thr Ala Met
                   85                  90                  95
   Tyr Tyr Cys Ala Arg Gly Gly Arg Asp Phe Gly Asp Ser Phe Asp Tyr
               100                 105                 110
   Trp Gly Gln Gly Thr Leu Val Thr Val Ser Ser Gly Gly Gly Gly Ser
               115                 120                 125
   Gly Gly Gly Gly Ser Gly Gly Gly Gly Ser Asp Val Val Met Thr Gln
       130                 135                 140
   Ser Pro Ser Phe Leu Ser Ala Phe Val Gly Asp Arg Ile Thr Ile Thr
   145                 150                 155                 160
   Cys Arg Ala Ser Ser Gly Ile Ser Arg Tyr Leu Ala Trp Tyr Gln Gln
                   165                 170                 175
   Ala Pro Gly Lys Ala Pro Lys Leu Leu Ile Tyr Ala Ala Ser Thr Leu
               180                 185                 190
   Gln Thr Gly Val Pro Ser Arg Phe Ser Gly Ser Gly Ser Gly Thr Glu
           195                 200                 205
   Phe Thr Leu Thr Ile Asn Ser Leu Gln Pro Glu Asp Phe Ala Thr Tyr
       210                 215                 220
   Tyr Cys Gln His Leu Asn Ser Tyr Pro Leu Thr Phe Gly Gly Gly Thr
   225                 230                 235                 240
   Lys Val Asp Ile Lys Arg Ala Ala
                   245

## Description

The present invention relates to the use of a composition in a method of manufacture of a medicament for the treatment of fungal infections, the composition comprising a novel antibody or antigen binding fragment thereof.

Fungal infections are a major cause of patient mortality in the intensive care unit and more generally in immunocompromised and debilitated patients (Gold, J.W.M., 1984, Am. J. Med. 76-:458463; Klein, R.S. et al., 1984, N. Engl. J. Med. 311: 354-357; Burnie, J.P., 1997, Current Anaesthesia & Critical Care 8: 180-183). The presence and persistence of fungal infections can be attributed to the selective pressure of broad-spectrum antifungals, frequently prolonged stay of patients in facilities such as an intensive care unit, problems in diagnosing the infections, and the lack of efficacy of the fungal agents used in therapy. While strict hygienic control may result in some prevention of fungal infections in a hospital or other environment, outbreaks of infections remain a serious problem and need to be addressed.

Systemic fungal infections such as invasive candidiasis and invasive aspergillosis may be caused by a variety of fungal pathogens, for example the virulent *Candida* species *C*. *albicans, C. tropicalis* and *C. krusei* and the less virulent species *C. parapsilosis* and *Torulopsis glabrata* (the latter referred to in some texts as *Candida glabrata*). Although *C. albicans* was once the most common fungal isolate obtained from intensive care units, recent studies indicate that *C*. *tropicalis, C. glabrata, C. parapsilosis* and *C. krusei* now account for about half of such isolates (Pfaller, M.A. et al., 1998, J. Clin. Microbiol. 36: 1886-1889; Pavese, P. et al., 1999, Pathol. Biol. 46: 579-583). The rise of non-albicans species implies the emergence of *Candida* species resistant to conventional antifungal therapy (Walsh, T.J. et al., New Eng. J. Med. 340: 764-771).

Detection and diagnosis of the fungal pathogen responsible for an infection is critical for subsequent therapy because antifungal agents may be more effective against certain strains. GB2240979 and EP0406029 (herein incorporated by reference in their entirety) disclosed a fungal stress protein and antibody thereto which could be used in a sensitive and highly specific test for detection of fungal pathogens.

Traditionally, *C. albicans, C. tropicalis* and *C. parapsilosis* have been treated by the antifungal agent amphotericin B, regarded as the "gold standard" of systemic antifungal therapy (Burnie, J.P., 1997, *supra*). Unfortunately, amphotericin B is itself highly toxic and its use is tempered by side effects including chills, fever, myalgia or thrombophlebitis. Other antifungal agents include the oral azole drugs (miconazole, ketoconazole, itraconazole, fluconazole) and 5-fluorocytosine. However, fungal species such as *C*. *krusei* and *T. glabrata* are resistant to fluconazole, and these species often occur in patients where this drug has been administered prophylactically. Furthermore, fluconazole-resistant strains of *C*. *albicans* have been reported (Opportunistic Pathogens, 1997, 1: 27-31). Thus despite the recent advances made in therapeutic drugs such as fluconazole, itraconazole and systemic liposomal-based variants of amphotericin B (Burnie, J.P., 1997, *supra*), the need for effective agents for treatment of fungal infections remains acute.

The present invention addresses the above-identified need by providing the use of a compostion for the manufacture of a medicament that is a significant improvement over prior art fungal agents for the treatment of human or animal fungal infections. A novel antibody can be incorporated into the composition. The composition of the present invention comprises antibody which may bind one or more epitopes of a fungal stress protein, in combination with known antifungal agents. The inventors have found that, surprisingly, the efficacy of antifungal agents against fungal infections is significantly enhanced, allowing for either lower treatment dosages or more effective treatment at the same dose, which allows for reduction of unwanted side-effects. Furthermore, the medicament of the present invention allows for effective treatment of fungal infections which are inherently resistant to the fungal agent used in the composition.

According to the present invention there is provided the use of a composition comprising an antibody or an antigen binding fragment thereof specific for one or more epitopes of a fungal stress protein and an antifungal agent comprising at least one of the group consisting of a polyene antifungal agent and an echinocandin antifungal agent in a method of manufacture of a medicament for the treatment of fungal infections, wherein the fungus causing said fungal infection is resistant to said antifungal agent *per se.*

Also disclosed herein is a combined preparation for simultaneous, separate or sequential use in the treatment of fungal infections, comprising an antibody or an antigen binding fragment thereof specific for one or more epitopes of a fungal stress protein and an antifungal agent comprising at least one of the group consisting a polyene antifungal agent and an echinocandin antifungal agent wherein the fungus causing said fungal infection is resistant to said antifungal agent *per se.*

The antibody may be specific for a heat shock protein from a member of the *Candida* or *Torulopsis* genera. (The *Candida* and *Torulopsis* genera are generally deemed to be synonymous.) In particular, the antibody may be specific for the heat shock protein comprising hsp90 from *Candida albicans,* as described in GB2240979 and EP0406029.

The antibody or an antigen binding fragment thereof may be specific for the epitope comprising the sequence of SEQ ID NO:1.

Antibodies, their manufacture and uses are well known and disclosed in, for example, Harlow, E. and Lane, D., Antibodies: A Laboratory Manual, Cold Spring Harbor Laboratory Press, Cold Spring Harbor, New York. 1999.

The antibodies may be generated using standard methods known in the art. Examples of antibodies include (but are not limited to) polyclonal, monoclonal, chimeric, single chain, Fab fragments, fragments produced by a Fab expression library, and antigen binding fragments of antibodies.

Antibodies may be produced in a range of hosts, for example goats, rabbits, rats, mice, humans, and others. They may be immunized by injection with heat shock protein from the *Candida* genus, for example hsp90 from *C. albicans,* or any fragment or oligopeptide thereof which has immunogenic properties. Depending on the host species, various adjuvants may be used to increase an immunological response. Such adjuvants include, but are not limited to, Freund's, mineral gels such as aluminum hydroxide, and surface active substances such as lysolecithin, pluronic polyols, polyanions, peptides, oil emulsions, keyhole limpet hemocyanin, and dinitrophenol. Among adjuvants used in humans, BCG (Bacille Calmette-Guerin) and *Corynebacterium parvum* are particularly useful.

Monoclonal antibodies to the heat shock protein from the *Candida* genus, for example hsp90 from *C*. *albicans,* or any fragment or oligopeptide thereof may be prepared using any technique which provides for the production of antibody molecules by continuous cell lines in culture. These include, but are not limited to, the hybridoma technique, the human B-cell hybridoma technique, and the EBV-hybridoma technique (Koehler et al., 1975, Nature, 256: 495-497; Kosbor et al., 1983, Immunol. Today 4: 72; Cote et al., 1983, PNAS USA, 80: 2026-2030; Cole et al., 1985, Monoclonal Antibodies and Cancer Therapy, Alan R. Liss Inc., New York, pp. 77-96).

In addition, techniques developed for the production of "chimeric antibodies", the splicing of mouse antibody genes to human antibody genes to obtain a molecule with appropriate antigen specificity and biological activity can be used (Morrison et al., 1984, PNAS USA, 81: 6851-6855; Neuberger et al., 1984, Nature, 312: 604-608; Takeda et al., 1985, Nature, 314: 452-454). Alternatively, techniques described for the production of single chain antibodies may be adapted, using methods known in the art, to produce *Candida* heat shock protein-specific single chain antibodies. Antibodies with related specificity, but of distinct idiotypic composition, may be generated by chain shuffling from random combinatorial immunoglobin libraries (Burton, D.R., 1991, PNAS USA, 88: 11120-11123).

Antibodies may also be produced by inducing *in vivo* production in the lymphocyte population or by screening recombinant immunoglobulin libraries or panels of highly specific binding reagents (Orlandi et al., 1989, PNAS USA, 86: 3833-3837; Winter, G. et al., 1991, Nature, 349: 293-299).

Antigen binding fragments may also be generated, for example the F(ab')2 fragments which can be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity (Huse et al., 1989, Science, 256: 1275-1281).

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between the heat shock protein from the *Candida* genus, for example hsp90 from *C. albicans,* or any fragment or oligopeptide thereof and its specific antibody. A two-site, monoclonal-based immunoassay utilizing monoclonal antibodies specific to two non-interfering *Candida* heat shock protein epitopes may be used, but a competitive binding assay may also be employed (Maddox et al., 1983, J. Exp. Med., 158: 1211-1216).

The antibody may comprise the sequence of SEQ ID NO: 2.

The polyene antifungal agent may, for example, comprise amphotericin B, a derivative of amphotericin B, or nystatin. Derivatives of amphotericin B include formulations such as AmBisome (supplied for example by NexStar Pharmaceuticals, Cambridge, UK), amphotericin-B lipid complex (Abelcet), amphotericin-B colloidal dispersion (Amphocil) and amphotericin-B intralipid emulsion (Burnie, J.P., 1997, *supra*), may be used. Amphotericin B may be used in combination with another antifungal agent, 5-fluorocytosine (Burnie, J.P., 1997, *supra*).

The echinocandin antifungal agent may, for example, be Anidulafungin (LY303366; Eli Lilly & Co., Indianapolis, USA). Echinocandins are cyclic lipopeptides that inhibit synthesis of β-1,3-glucan in fungi (Redding, J.A. et al., 1998, Antimicrob. Agents Chemo. Ther. 42(3): 1187-1194).

The fungal infection which may be treated by the composition or combined preparation may be Mucormycosis, Blastomycosis, Coccidioidomycosis or Paracoccidioidomycosis, or the fungal infection may be caused by a Candida, Cryptococcus, Histoplasma, Aspergillus, or Torulopsis organism. The term "Coccidioidomycosis" is also referred to in the field as "Coccidiomycosis", and the term "Paracoccidioidomycosis" is likewise synonymous with "Paracoccidiomycosis". The fungal infection is resistant to the antifungal agent *per se,* ie. fungal infections which are intrinsically untreatable by specific agents because that specific antifungal agent is ineffective as traditionally utilised on its own.

There is disclosed herein a method of manufacture of a medicament for the treatment of fungal infections of the human or animal body characterised in the use of a composition or combined preparation as described in the present application. Methods of manufacture of medicaments are well known. For example, a medicament may additionally comprise a pharmaceutically acceptable carrier, diluent or excipient (Remington's Pharmaceutical Sciences and US Pharmacopoeia, 1984, Mack Publishing Company, Easton, PA, USA).

Also disclosed is a method of treatment of fungal infections of the human or animal body comprising administering a composition or combined preparation according to the present application to a patient in need of same. The exact dose (i.e. a pharmaceutically acceptable dose) of the composition or combined preparation to be administered to a patient may be readily determined by one skilled in the art, for example by the use of simple dose-response experiments. The composition or combined preparation may be administered orally.

Further disclosed is a kit comprising an antibody comprising the sequence according to SEQ ID NO: 2 or an antigen binding fragment thereof specific for one or more epitopes of a fungal stress protein and an antifungal agent comprising any one of the group consisting a polyene antifungal agent and an echinocandin antifungal agent. The kit may be for use in the treatment of fungal infections, wherein the fungus causing the fungal infection is resistant to the antifungal agent *per se.*

The antibody disclosed herein may have a diagnostic use. Thus for diagnostic use the antibody may be employed to detect whether the stress protein is present in a host organism, to confirm whether the host has a particular fungal infection, for example Mucormycosis, Blastomycosis, Coccidioidomycosis or Paracoccidioidomycosis, or an infection due to a Candida, Cryptococcus, Histoplasma, Aspergillus, or Torulopsis organism, or for example in the diagnosis of fungal abscesses, especially hepatic Candidiasis, and/or to monitor the progress of therapeutic treatment of such infections. Diagnostic methods of this type may generally employ standard techniques, for example immunological methods such as enzyme-linked immunosorbent methods, radioimmuno-methods, latex agglutination methods or immunoblotting methods.

The antibody disclosed herein may be labelled with a detectable label or may be conjugated with effector molecule for example a drug e.g. an anti-fungal agent such as amphotericin B or fluorocytosine or a toxin, such as ricin, or an enzyme, using conventional procedures and the disclosure extends to such labelled antibodies or antibody conjugates.

Also disclosed herein is the use of the antibody or antigen binding fragment in the preparation of a diagnostic for diagnosing one or more fungal infections. The diagnostic may be provided in a kit. The kit may include instructions for use in diagnosing one or more fungal infections.

If desired, mixtures of antibodies may be used for diagnosis or treatment, for example mixtures of two or more antibodies recognising different epitopes of a fungal stress protein according to the invention, and/or mixtures of antibodies of a different class, e.g. mixtures of IgG and IgM antibodies recognising the same or different epitope(s) of the invention.

The present invention will be further apparent from the following description, which shows, by way of example only, specific embodiments of the composition and experimentation therewith.

### EXPERIMENTAL

Experiments described below investigated the antifungal effect of antibody against an hsp90 antigen derived from *Candida albicans* used in combination with antifungals such as amphotericin B or fluconazole. Results show that, in some cases, the combination of antibody and antifungal agent causes an enhanced antifungal effect compared with each of the compounds on their own. A surprisingly strong synergistic effect is demonstrated for amphotericin B in combination with anti-*Candida albicans* hsp90 antibody against a variety of common problematic fungal pathogens. This synergistic effect has significant implications for clinical treatment of fungal infections. A preliminary clinical study involving four patients suffering from *Candida* infections demonstrated the effectiveness of the present invention for humans.

### Material and Methods

### Strains:

Non *Aspergillus* yeast strains used (Table 1) were plated onto Sabouraud's dextrose agar (Oxoid, Basingstoke, UK) and incubated at 37°C for 24 hours. The strains were identified with the API 20C system (BioMerieux, Marcy L'Etoile, France). If needed, microscopical examinations of morphology on cornmeal agar (Oxoid) was used to confirm the identity.

Isolates of *Aspergillus spp* (Table 1) were grown on Sabouraud's dextrose agar (Oxoid, Basingstoke, UK) at 35°C for 24 hours.

| **Table 1. Origin of strains** | |
|---|---|
| **Strain** | **Reference** |
| 1. *Candida albicans* | B.M.J., 1985, 290: 746-748 |
| (outbreak) | |
| 2. *C. albicans* | Opportunistic Pathogens, 1997, 1: 27-31 |
| (Fluconazole resistant) | |
| 3. *C*. *krusei* | Int. J. Systemic Bacteriol., 1996, 46: 35-40 |
| (FA/157) | |
| 4. *C*. *tropicalis* | National Collection of Pathogenic Fungi |
| (NCPF #3111) | |
| 5. *C. parapsilosis* | National Collection of Pathogenic Fungi |
| (NCPF #3104) | |
| 6. *Torulopsis glabrata* | National Collection of Pathogenic Fungi |
| (NCPF #3240) | |
| 7. *Aspergillus fumigatus* | National Collection of Pathogenic Fungi |
| (NCPF#2109) | |
| 8. *Aspergillus flavus* | Clinical isolate, identified by characteristic morphology |
| 9. *Aspergillus niger* | Clinical isolate, identified by characteristic morphology |

### Inoculum:

For non-*Aspergillus* strains, suspensions were prepared from individual colonies (diameter ≥ 1mm) in 5 ml of sterile 0.85% saline to a density of 1 x 10⁴ cells/ml as established by counting on a haemocytometer grid. For *Aspergillus* strains, see below.

### Antifungal agents:

Amphotericin B was purchased from Sigma (Poole, Dorset) as a lyophilized powder for intravenous administration (Fungizone). Fluconazole was supplied as a solution for intravenous administration (Diflucan) by Pfizer. Amphotericin B was dissolved in dimethyl sulphoxide at a concentration of 1.2 mg/ml and fluconazole was dissolved in 0.85% saline also at a concentration of 1.2 mg/ml. Stock solutions were stored at -70°C until used. Abelcet (liposomal amphotericin B) manufactured by Bristol-Meyers Squib (USA) and prepared according to the manufacturers guidelines was used in the clinical study.

### Antibody:

The DNA sequence of a former antibody specific for the *Candida albicans* hsp90 epitope disclosed in GB2240979 and EP0406029 was genetically modified by codon optimisation for expression in *Escherichia coli* (Operon Technologies Inc., Alameda, CA, USA) and inserted into an *E. coli* expression vector. The amino acid sequence of the anti-hsp90 antibody of the present invention comprises the sequence of SEQ ID NO: 2 (includes the heavy, light and spacer domains). The antibody according to the present invention recognises the epitope comprising the sequence of SEQ ID NO: 1.

The anti-hsp90 antibody was expressed in an *Escherichia coli* host and then purified by affinity chromatography and an imidazole exchange column up to 95 % purity. Standard molecular biology protocols were employed (see, for example, Harlow & Lane, *supra;* Sambrook, J. et al., 1989, Molecular Cloning: A Laboratory Manual, 2nd Edition, Cold Spring Harbor Laboratory Press, Cold Spring Harbor, New York; Sambrook, J. & Russell, D., 2001, Molecular Cloning: A Laboratory Manual, 3rd Edition, Cold Spring Harbor Laboratory Press, Cold Spring Harbor).

Formulations of Mycograb (RTM) were prepared as follows: a vial containing 10 mg of pure anti-hsp90 antibody, 150 mg of pharmaceutical grade (Ph Eur) Urea and 174 mg L-Arginine (Ph Eur) were reconstituted in 5 ml water..

### Assay media:

RPMI broth was prepared from RPMI 1640 broth medium (Sigma R7880) supplemented with 0.3 g of glutamine per litre, buffered with 34.6 g of morpholine propanesulfonic acid (MOPS) per litre and adjusted to ph 7.0.

### The Broth microdilution test:

Twofold dilutions (40 to 0.024 mg/ml for amphotericin B and 400 to 0.4 mg/ml for fluconazole) were prepared in RPMI broth starting from the two stock solutions. A 100 µl suspension of the inoculum diluted 1 in 10 (equivalent to 1 x 10³ cfu) was added to the microtiter plates. To this was added 50 µl of the antifungal and then 50 µl of the antibody. Antibody was either neat (0.4 mg/ml), or diluted to 1/10 or 1/100. When antibody was absent, the 50 µl volume was made up by RPMI. The total volume in each well was 200 µl. Final concentrations of antibody in the experiments were: 100 µg/ml ("neat"), 10 µg/ml ("1/10 antibody") or 1 µg/ml ("1/100 antibody").

Plates were incubated at 37°C overnight and the minimum inhibitory concentration (MIC) defined by the lower concentration inhibiting growth.

Colony counts were determined for the wells where there was a visual reduction in yeast growth. Results were represented as colony forming units per ml of broth (cfu/ml).

### Aspergillus Studies:

Isolates of *Aspergillus fumigatus, Aspergillus flavus* and *Aspergillus niger* were prepared in RPMI 1640 medium. The suspensions were prepared to give a final inoculum of 2x10⁴ conidia per ml and these were dispensed in 100 µl aliquots into flat-bottomed microtitre plates. A double dilution series of Amphotericin B ranging from 250 µg/ml to 0.75 µg/ml was prepared and dispensed to the appropriate wells. Mycograb was added to each of the wells at a final concentration of 100 µg/ml in formulation buffer. A control series was also prepared for each isolate which contained formulation buffer only. The plates were then incubated at 35°C/200 rpm for 48 hours and MIC values for each isolate were determined by absence or presence of growth wells.

### Animal Synergy:

Thirty CD1 mice (each weighing about 25 g) were injected with 100 µl of *C. albicans* outbreak strain (equivalent to 1.5 x 10⁷ cfu) and after 2 hours the mice were split into three groups and injected with:
(A) Group 1- -100 µl solution of 10 mM ammonium acetate (AAT; pH 9), followed by 100 µl amphotericin B equivalent to 0.6 mg/kg in a 5% (w/v) glucose solution;
(B) Group 2 - 100 µl solution of 10 mM AAT (pH 9) solution containing 500 µg anti-hsp90 antibody, followed by 100 µl amphotericin B equivalent to 0.6 mg/kg in a 5 % (w/v) glucose solution; and
(C) Group 3 - 100 µl solution of 10 mM AAT (pH 9) containing 50 µg anti-hsp90 antibody, followed by 100 µl amphotericin B equivalent to 0.6 mg/kg in a 5% (w/v) glucose solution.

The animals were culled after 48 hours and yeast counts on Sabouraud's plates of liver, spleen and kidney tissue performed.

### Clinical study:

An open-label safety and pharmacokinetics study of the anti-hsp90 antibody (in the form of Mycograb; see above) involving four patients suffering from *Candida* infections was conducted at the Central Manchester Health Care Trust Hospital and the Wythenshawe Hospital, both in Manchester, UK. Patients were examined for the signs of sepsis due to *Candida,* including: positive cultures of *C*. *albicans* from multiple or deep sites; high or swinging temperature (pyrexia); high pulse rate (tachycardia) and high white cell count ("WBC").

Following conventional treatment with Abelcet (liposomal amphotericin B) and/or fluconazole, the patients were then additionally given various doses of Mycograb, including an optional test dose (0.1 mg/kg), and therapeutic dose(s) of 1 mg/kg. Patients were monitored for clinical and laboratory signs of infection (laboratory parameters tested include blood chemistry, haematology and clotting factors) and serum and urine levels of Mycograb tested.

### Results

*In vitro* experiments examining the effect of combining an anti-*Candida albicans* hsp90 antibody ("antibody") and antifungal agents are presented in Tables 2 to 18. Animal experimental results are presented in Table 19.

### In vitro experiments:

### Compositions containing antibody and fluconazole:

Table 2 shows the minimum inhibitory concentrations (MICs) of fluconazole against the test fungal pathogens, with or without the presence of the anti-*C*. *albicans* hsp90 antibody at different dilutions, as assessed by the Broth microdilution test. In the presence of neat antibody and antibody diluted 10-fold, the MIC for the outbreak strain of *C. albicans* was reduced four-fold (1.56 µg/ml to 0.39 µg/ml fluconazole), whereas a 100-fold dilution of the antibody resulted in a two-fold reduction of fluconazole MIC.

A slight reduction in fluconazole MIC was observed for the fluconazole resistant strain of *C*. *albicans* and *C*. *krusei* in the presence of neat antibody. At a dilution of 1/10 and 1/100, however, the antibody had no effect on the fluconazole MIC of these strains in comparison with no antibody.

For the remaining fungal strains, ie. *C*. *tropicalis, C. parapsilosis* and *T. glabrata,* the anti-*C*. *albicans* hsp90 antibody had no discernable effect on the fluconazole MICs.

| **Table 2. MICs to fluconazole** | | | | |
|---|---|---|---|---|
| **MIC (µg/ml)** | | | | |
| | Fluconazole | Fluconazole | Fluconazole | Fluconazole |
| | No antibody | Neat antibody | 1/10 antibody | 1/100 antibody |
| | | [100 µg/ml] | [10µg/ml] | [1µg/ml] |
| *C. albicans* Outbreak strain | 1.56 | 0.39 | 0.39 | 0.78 |
| *C. albicans* Fluconazole Resistant | 25 | 12.5 | 25 | 25 |
| *C. krusei* | 100 | 50 | 100 | 100 |
| *C. tropicalis* | 3.125 | 3.125 | 3.125 | 3.125 |
| *T. glabrata* | 1.56 | 1.56 | 1.56 | 1.56 |
| *C. parapsilosis* | 6.25 | 6.25 | 6.25 | 6.25 |

Further experiments which quantified the number of cell colonies surviving at different fluconazole concentrations with different dilutions of the anti-*C. albicans* hsp90 antibody were undertaken for each of the fungal strains represented in Table 2.

At the fluconazole concentrations examined, the survival rate of *C*. *albicans* (outbreak strain) was not reduced by the addition of neat antibody or antibody diluted 100-fold (Table 3).

| **Table 3. Colony counts (in cfu/ml) for *C. albicans* (outbreak strain) against fluconazole** | | | |
|---|---|---|---|
| | **Fluconazole concentration (µg/ml)** | | |
| | 0.09 | 0.19 | 0.39 |
| No antibody | 3.6 x 10⁵ | 1 x 10⁵ | 5 x 10⁴ |
| | | | |
| Neat antibody | 3.6 x 10⁶ | 1.3 x 10⁵ | 1.3 x 10⁴ |
| [100 µg/ml] | | | |
| 1/100 antibody | | | |
| [1 µg/ml] | 1 x 10⁶ | 2.6 x 10⁴ | 5.3 x 10⁴ |
| | | | |
| (Control 6 x 10⁶ cfu/ml) | | | |

For the fluconazole-resistant strain of *C*. *albicans,* a two-fold reduction in colony survival was observed at 12.5 µg/ml fluconazole in the presence of neat antibody (Table 4). Slight reductions in the survival rate of this strain were noticed at lower concentrations of fluconazole in the presence of neat antibody, but no effect was discernable at a 1/100 dilution of the antibody.

| **Table 4. Colony counts (in cfu/ml) for the fluconazole resistant strain of *C***. ***albicans* against fluconazole** | | | | |
|---|---|---|---|---|
| | **Fluconazole concentration (µg/ml)** | | | |
| | 1.56 | 3.12 | 6.25 | 12.5 |
| No antibody | 3 x 10⁷ | 1.3 x 10⁷ | 3 x 10⁶ | 6 x 10⁶ |
| | | | | |
| Neat antibody | 2 x 10⁶ | 4.3 x 10⁵ | 5.6 x 10⁴ | 6 x 10³ |
| [100 µg/ml] | | | | |
| | | | | |
| 1/100 antibody | 3 x 10⁷ | 1.1 x 10⁷ | 1.1 x 10⁷ | 6.3 x 10⁶ |
| [1 µg/ml] | | | | |
| | | | | |
| (Control 2.6 x 10⁷ cfu/ml) | | | | |

Neat or diluted antibody had no significant antifungal effect against *C*. *krusei* at the fluconazole concentrations tested (Table 5).

| **Table 5. Colony counts (in cfu/ml) for *C*. *krusei* against fluconazole** | | |
|---|---|---|
| | **Fluconazole concentration (µg/ml)** | |
| | 25 | 50 |
| No antibody | 3.2 x 10⁷ | 1.6 x 10⁷ |
| | | |
| Neat antibody | 8.3 x 10⁶ | 6 x 10⁶ |
| [100 µg/ml] | | |
| | | |
| 1/100 antibody | 1.3 x 10⁶ | 1.6 x 10⁶ |
| [1 µg/ml] | | |
| | | |
| (Control 1 x 10⁷ cfu/ml) | | |

For *C. tropicalis,* no marked effect on survival rate could be seen for each of the fluconazole concentrations examined in the presence or absence of antibody (Table 6).

| **Table 6. Colony counts (in cfu/ml) for *C*. *tropicalis* against fluconazole** | | | |
|---|---|---|---|
| | **Fluconazole concentration (µg/ml)** | | |
| | 0.09 | 0.19 | 0.39 |
| No antibody | 5 x 10⁵ | 6 x 10³ | 6 x 10² |
| | | | |
| Neat antibody | 7 x 10⁵ | 6.3 x 10⁴ | 9 x 10² |
| [100 µg/ml] | | | |
| | | | |
| 1/100 antibody | 1 x 10⁵ | 6 x 10³ | 2 x 10³ |
| [1 µg/ml] | | | |
| | | | |
| (Control 1.6 x 10⁶ cfu/ml) | | | |

Table 7 shows that presence or absence of the antibody had no effect of the survival rate of *T. glabrata* colonies at each of the fluconazole concentrations tested.

| **Table 7. Colony counts (in cfu/ml) for *T. glabrata* against fluconazole** | | | |
|---|---|---|---|
| | **Fluconazole concentration (µg/ml)** | | |
| | 0.39 | 0.78 | 1.56 |
| No antibody | 2 x 10⁷ | 1 x 10⁷ | 6 x 10⁴ |
| | | | |
| Neat antibody | 1.5 x 10⁷ | 1.2 x 10⁷ | 9.3 x 10⁵ |
| [100 µg/ml] | | | |
| | | | |
| 1/100 antibody | 2.3 x 10⁷ | 1.9 x 10⁷ | 2 x 10⁵ |
| [1 µg/ml] | | | |
| | | | |
| (Control 4.3 x 10⁷ cfu/ml) | | | |

Presence or absence of the antibody had no notable effect on the survival rate of *C*. *parapsilosis* colonies at the fluconazole concentrations as indicated in Table 8.

| **Table 8. Colony counts (in cfu/ml) for *C. parapsilosis* against fluconazole** | | | | |
|---|---|---|---|---|
| | **Fluconazole concentration (µg/ml)** | | | |
| | 0.78 | 1.56 | 3.13 | 6.25 |
| No antibody | 7 x 10⁶ | 5.6 x 10⁶ | 2.6 x 10⁶ | 3 x 10⁶ |
| | | | | |
| Neat antibody | 8.6 x 10⁶ | 2.3 x 10⁶ | 1.6 x 10⁶ | 1.6 x 10⁶ |
| [100 µg/ml] | | | | |
| | | | | |
| 1/100 antibody | 4 x 10⁵ | 3 x 10⁶ | 2.3 x 10⁶ | 5 x 10⁶ |
| [1 µg/ml] | | | | |
| | | | | |
| (Control 1 x 10⁷ cfu/ml) | | | | |

### Compositions with antibody and amphotericin B:

Table 9 shows the minimum inhibitory concentrations (MICs) of amphotericin B against the test fungal pathogens, with or without the presence of the anti-*C. albicans* hsp90 antibody at different dilutions, as assessed by the Broth microdilution test.

In contrast with the results obtained for fluconazole (see Tables 2-8 *supra*), all strains tested here showed at least a four-fold drop in MIC of amphotericin B when undiluted antibody was added to the incubation broth (Table 9). Furthermore, in all strains examined, there was at least a two-fold drop in amphotericin B MIC even when the antibody diluted 100-fold was added to the incubation broth (final antibody concentration: 1 µg/ml).

The greatest effect of the composition comprising antibody and amphotericin B at reducing the MIC of amphotericin B was observed with the fluconazole resistant strain of *C. albicans.* Neat antibody yielded a ten-fold reduction in the amphotericin B MIC, and even at a 100-fold antibody dilution, the amphotericin B MIC was reduced by approximately 25 % (Table 9).

| **Table 9. MICs to amphotericin B** | | | | |
|---|---|---|---|---|
| **MIC (µg/ml)** | | | | |
| | Amphotericin | Amphotericin | Amphotericin | Amphotericin |
| | No antibody | Neat antibody | 1/10 antibody | 1/100 antibody |
| | | [100 µg/ml] | [10 µg/ml] | [1 µ/ml] |
| *C. albicans* | 0.156 | 0.039 | 0.039 | 0.078 |
| (outbreak) | | | | |
| | | | | |
| *C. albicans* | 0.312 | 0.039 | 0.078 | 0.078 |
| Fluconazole Resistant | | | | |
| | | | | |
| *C*. *krusei* | 0.625 | 0.156 | 0.312 | 0.312 |
| | | | | |
| *C. tropicalis* | 0.078 | 0.019 | 0.039 | 0.039 |
| | | | | |
| *T. glabrata* | 0.039 | <0.009 | <0.009 | 0.019 |
| | | | | |
| *C. parapsilosis* | 0.625 | 0.156 | 0.313 | 0.313 |

Detailed experiments which quantified the number of cell colonies surviving at different amphotericin B concentrations with different dilutions of the anti-*C*. *albicans* hsp90 antibody were undertaken for each of the fungal strains represented in Table 9.

Table 10 shows survival rates for the outbreak strain of *C*. *albicans* incubated with amphotericin B in the presence or absence of antibody. A dramatic reduction (at least 10-fold) in the number of surviving colonies was effected by the antibody at all the amphotericin concentrations tested. For example, at 0.078 µg/ml amphotericin B, the survival rate of *C*. *albicans* (outbreak strain) was 0.2% in the presence of antibody diluted 100-fold compared with the survival rate of the strain without antibody. The inhibitory effect of the antibody diluted 100-fold at 0.078 µg/ml amphotericin B was equivalent to the survival rate of this strain at 0.156 µg/ml amphotericin B (without antibody). Therefore, even very diluted antibody is able to effect a reduction in the amount of amphotericin B required to achieve a specific mortality rate in this strain.

| **Table 10. Colony counts (in cfu/ml) for the outbreak strain of *C. albicans* against amphotericin B** | | | | |
|---|---|---|---|---|
| | **Amphotericin B concentration (µg/ml)** | | | |
| | 0.019 | 0.039 | 0.078 | 0.156 |
| No antibody | 1 x 10⁷ | 1.6 x 10⁷ | 4.1 x 10⁵ | 1.3 x 10³ |
| | | | | |
| Neat antibody | 5.3 x 10⁵ | 6 x 10³ | 3 x 10² | 4.3 x 10² |
| [100 µg/ml] | | | | |
| | | | | |
| 1/10 antibody | 5 x 10⁵ | 1 x 10⁴ | 3.0 x 10² | 3 x 10¹ |
| [10 µg/ml] | | | | |
| | | | | |
| 1/100 antibody | 6.6 x 10⁶ | 3.2 x 10⁵ | 8.0 x 10² | 1 x 10² |
| [1 µg/ml] | | | | |
| | | | | |
| (Control 1 x 10⁷ cfu/ml) | | | | |

Table 11 shows the survival rate of colonies of *C. albicans* (fluconazole resistant strain) at different concentrations of amphotericin B and at different antibody dilutions. No noticeable effect of the antibody or amphotericin B could be seen at lower concentrations of the antifungal agent. However, at amphotericin B levels approaching the MIC of the antifungal agent (see Table 9, *supra*), the antibody was observed to have a marked effect on colony survival. For example, at 0.078 µg/ml amphotericin B, antibody at a 100-fold dilution effected a survival rate of this strain of 0.1 % compared with no antibody.

| **Table 11. Colony counts (in cfu/ml) for the fluconazole resistant strain of *C*. *albicans* against amphotericin B** | | | |
|---|---|---|---|
| | **Amphotericin B concentration (µg/ml)** | | |
| | 0.019 | 0.039 | 0.078 |
| No antibody | 4.6 x 10⁶ | 4.3 x 10⁶ | 6 x 10⁶ |
| | | | |
| Neat antibody | 5.3 x 10⁵ | 3.0 x 10³ | 3 x 10³ |
| [100 µg/ml] | | | |
| | | | |
| 1/10 antibody | 2.2 x 10⁶ | 6.3 x 10⁴ | 1.6 x 10³ |
| [10 µg/ml] | | | |
| | | | |
| 1/100 antibody | 3.4 x 10⁶ | 1.6 x 10⁵ | 5.3 x 10³ |
| [1 µg/ml] | | | |
| | | | |
| (Control 1.6 x 10⁷ cfu/ml) | | | |

Colony survival rates of *C*. *krusei* in the presence of amphotericin B and different amounts of antibody are represented in Table 12. The antibody can be seen to be very effective against this strain at the higher concentrations of amphotericin B examined. Even at a 100-fold dilution, the number of *C*. *krusei* colonies detected in the presence of 0.312 µg/ml amphotericin B was 0.01 % of those surviving without antibody.

| **Table 12. Colony counts (in cfu/ml) for *C. krusei* against amphotericin B** | | | |
|---|---|---|---|
| | Amphotericin B concentration (µg/ml) | | |
| | 0.078 | 0.156 | 0.312 |
| No antibody | 1 x 10⁷ | 1.7 x 10⁷ | 9 x 10⁵ |
| | | | |
| Neat antibody | 1 x 10⁶ | 1.76 x 10⁵ | <10² |
| [100 µg/ml] | | | |
| | | | |
| 1/10 antibody | 6.3 x 10⁶ | 1.6 x 10⁵ | <10² |
| [10 µg/ml] | | | |
| | | | |
| 1/100 antibody | 1.6 x 10⁷ | 1.53 x 10⁶ | 1.3 x 10² |
| [1 µg/ml] | | | |
| | | | |
| (Control 1 x 10⁷ cfu/ml) | | | |

For all concentrations of amphotericin B tested (range from 0.019 - 0.156 µg/ml), the antibody was seen to be effective at reducing the colony survival rate of the strain *C. tropicalis* (Table 13). The effect was enhanced at higher antibody and amphotericin B concentrations.

| **Table 13. Colony counts (in cfu/ml) for *C*. *tropicalis* against amphotericin B** | | | | |
|---|---|---|---|---|
| | **Amphotericin concentration (µg/ml)** | | | |
| | 0.019 | 0.039 | 0.078 | 0.156 |
| No antibody | 1.3 x 10⁶ | 1 x 10⁶ | 2.6 x 10⁵ | 6.6 x 10³ |
| | | | | |
| Neat antibody | 1.1 x 10⁴ | 2.3 x 10⁴ | 2 x 10² | 0 |
| [100 µg/ml] | | | | |
| | | | | |
| 1/10 antibody | 1 x 10⁴ | 3.4 x 10⁴ | 4 x 10² | 3 x 10¹ |
| [10 µg/ml] | | | | |
| | | | | |
| 1/100 antibody | 1.1 x 10⁶ | 2 x 10⁴ | 2.6 x 10³ | 0 |
| [1 µg/ml] | | | | |
| | | | | |
| (Control 1.6 x 10⁶ cfu/ml) | | | | |

Table 14 shows the survival rate for *T. glabrata* in the presence of various concentrations of amphotericin B and antibody. The antibody was observed to be highly effective at inhibiting growth of *T. glabrata* at all concentrations of amphotericin B tested. For example, at a 100-fold dilution of the antibody, the growth of this strain was inhibited by 99.2 % at 0.009 µg/ml amphotericin B, 99.99% at 0.019 µg/ml amphotericin B and 99.91 % at 0.039 µg/ml amphotericin B.

| **Table 14. Colony counts (in cfu/ml) for *T. glabrata* against amphotericin B** | | | |
|---|---|---|---|
| | Amphotericin B concentration (µg/ml) | | |
| | 0.009 | 0.019 | 0.039 |
| No antibody | 1.1 x 10⁷ | 9.6 x 10⁶ | 1.4 x 10⁵ |
| | | | |
| Neat antibody | 8.6 x 10³ | 8.3 x 10² | 2.6 x 10² |
| [100 µg/ml] | | | |
| | | | |
| 1/10 antibody | 9 x 10⁵ | 6.3 x 10³ | 2.3 x 10² |
| [10 µg/ml] | | | |
| | | | |
| 1/100 antibody | 9 x 10⁴ | 1 x 10³ | 1.3 x 10² |
| [1 µg/ml] | | | |
| | | | |
| (Control 1 x 10⁷ cfu/ml) | | | |

The survival of the fungal strain *C. parapsilosis* at different levels of antibody and amphotericin B is shown in Table 15. Neat antibody was observed to achieve a reduction in the survival rate of this strain at all levels of amphotericin B tested. At lower concentrations of antibody, the effect was less dramatic than for the other strains examined.

| **Table 15. Colony counts (in cfu/ml) for *C. parapsilosis* against amphotericin B** | | | |
|---|---|---|---|
| | **Amphotericin B concentration (µg/ml)** | | |
| | 0.156 | 0.313 | 0.626 |
| No antibody | 1.46 x 10⁷ | 3 x 10⁶ | 6.3 x 10³ |
| | | | |
| Neat antibody | 1.3 x 10⁵ | 3 x 10⁴ | 6.0 x 10² |
| [100 µg/ml] | | | |
| | | | |
| 1/10 antibody | | | |
| [10 µg/ml] | 1.03 x 10⁷ | 1.76 x 10⁵ | 6.0 x 10³ |
| | | | |
| 1/100 antibody | 1.8 x 10⁷ | 2.9 x 10⁵ | 3.3 x 10³ |
| [1 µg/ml] | | | |
| | | | |
| (Control 1 x 10⁷ cfu/ml) | | | |

### Compositions with antibody but without antifungal agent:

In a further experiment, the effect of different concentrations of the anti-*C*. *albicans* hsp90 antibody alone (no antifungal agent) on the different fungal strains (used in Tables 2-15, *supra*) was tested. The results shown in Table 16 reveal that for the most of the strains used, the antibody itself had no effect on their survival. However, some diminution in survival rate which can be attributed to the antibody alone was observed in the strains *T*. *glabrata, C. tropicalis* and *C*. *parapsilosis.*

| **Table 16. Effect of antibody on its own against yeast growth (expressed as cfu/ml)** | | | | | | |
|---|---|---|---|---|---|---|
| Antibody | *C. albicans* | *C. albicans* | *C*. *krusei* | *T. glabrata* | *C. tropicalis* | *C. parapsilosis* |
| (µg/ml) | (outbreak) | (fluconazole resistant) | | | | |
| 0 | 1.2 x 10⁷ | 1 x 10⁷ | 3.3 x 10⁷ | 1.3 x 10⁷ | 1 x 10⁶ | 7.0 x 10⁶ |
| | | | | | | |
| 0.313 | 5.6 x 10⁶ | 6 x 10⁶ | 1.6 x 10⁶ | 1.2 x 10⁷ | 6.0 x 10⁵ | 2.6 x 10⁴ |
| | | | | | | |
| 0.625 | 3.3 x 10⁶ | 5.3 x 10⁶ | 9.3 x 10⁶ | 1 x 10⁷ | 3.3 x 10⁵ | 3.0 x 10⁴ |
| | | | | | | |
| 1.25 | 5.0 x 10⁶ | 5.6 x 10⁶ | 6.6 x 10⁶ | 6.6 x 10⁶ | 3.6 x 10⁵ | 1.6 x 10⁴ |
| | | | | | | |
| 2.5 | 5.3 x 10⁶ | 6.3 x 10⁶ | 4.3 x 10⁶ | 6 x 10⁵ | 9 x 10⁴ | 6.6 x 10³ |

### Experiments with Aspergillus spp:

The MIC of *Aspergillus fumigatus* to Amphotericin B was 2.5 µg/ml. With the addition of Mycograb, the MIC shifted to 0.125 µg/ml (two-fold decrease). The MIC of *Aspergillus flavus* to Amphotericin B was 2.5 µg/ml. With the addition of 100 µg/ml of Mycograb the MIC shifted to 0.125 µg/ml (two-fold decrease). The MIC of *Aspergillus niger* to Amphotericin B was 2.5 µg/ml. With addition of 100 µg/ml of Mycograb the MIC shifted to 0.125 µg/ml (two-fold decrease).

### Summary of in vitro results:

The results shown in Tables 2-16 reveal that while the anti-*C*. *albicans* hsp90 antibody on its own was able to inhibit growth of certain fungal strains, a surprisingly high level of antifungal activity against all the strains examined was observed when the antibody was used in combination with amphotericin B. This surprising effect between the antibody and amphotericin B is not observed with other antifungal agents examined: fluconazole combined with the antibody did not produce a significant and potentially useful outcome.

Using the cut-off criterion of a four-fold difference in MIC improvement, data in Table 2 reveal that fluconazole combined with neat antibody (final concentration 100 µg/ml) or a 10-fold dilution of antibody (final concentration 10 µg/ml) was effective only against the outbreak strain of *C. albicans.* However, using the same criterion, it can be seen from Table 9 that amphotericin B combined with neat antibody or a 10-fold dilution of the antibody was effective against all the fungal strains tested. It can therefore be concluded that there is a strong synergy between amphotericin B and the anti-*C. albicans* hsp90 antibody as measured by improvement in MIC.

For the experiments in which fungal colonies were quantified for different antifungal and antibody treatments (see Tables 3-8 and 10-15, *supra*), a different cut-off criterion which defines a two log drop (100-fold) drop in surviving colonies can be employed to assess potentially useful combinations of treatments.

A summary of the results for fluconazole in combination with the anti-*C*. *albicans* hsp90 antibody is presented in Table 17. Here, the lowest concentration of fluconazole resulting in the desired effect (or the highest concentration used in the experiment) used is shown, together with an indication of the cut-off criterion of at least a 100-fold drop is fungal survival rate was achieved. The results show that only the fluconazole resistant strain of *C*. *albicans* when combined with fluconazole and neat antibody produced a significant effect.

| **Table 17. Summary of *in vitro* results for fluconazole** | | | |
|---|---|---|---|
| | Fluconazole | Neat Antibody | Table |
| | (µg/ml) | [100 µg/ml] | |
| *C. albicans* | 0.39 | - | 3 |
| Outbreak | | | |
| | | | |
| *C.albicans* | 12.5 | + | 4 |
| Fluconazole resistant | | | |
| | | | |
| *C*. *krusei* | 50 | - | 5 |
| | | | |
| *C. tropicalis* | 0.39 | - | 6 |
| | | | |
| *T. glabrata* | 1.56 | - | 7 |
| | | | |
| *C. parapsilosis* | 6.25 | - | 8 |
| | | | |
| (+ indicates cut-off criterion satisfied; - indicates cut-off criterion not satisfied; cut-off criterion is at least 100-fold reduction in colony count) | | | |

A summary of the results for amphotericin B in combination with the anti-*C*. *albicans* hsp90 antibody is presented in Table 18. It can be seen that the cut-off criterion (100-fold reduction in fungal colony growth) is satisfied with neat antibody for all fungal strains examined, with a 10-fold dilution of the antibody for *C*. *albicans* (outbreak strain and fluconazole resistant strain), *C*. *krusei* and *T. glabrata,* and with a 100-fold reduction in antibody for *C*. *albicans* (outbreak strain) and *T. glabrata.*

It is noteworthy that synergy between amphotericin B and the antibody was observed not only against fluconazole sensitive strains of *C*. *albicans* but also fluconazole resistant strains of *C*. *albicans* and yeasts such as *Candida krusei* and *T. glabrata* which are intrinsically resistant to fluconazoles.

| **Table 18. Summary of *in vitro* results for amphotericin B** | | | | | |
|---|---|---|---|---|---|
| | Amphotericin | Antibody | Antibody | Antibody | |
| | (µg/ml) | Neat | 1/10 | 1/100 | Table |
| | | [100 µg/ml] | [10 µg/ml] | [1 µg/ml] | |
| *C.albicans* | 0.039 | + | + | + | 10 |
| Outbreak | | | | | |
| | | | | | |
| *C.albicans* | 0.039 | + | + | - | 11 |
| Fluconazole resistant | | | | | |
| | | | | | |
| *C*. *krusei* | 0.156 | + | + | - | 12 |
| | | | | | |
| *C. tropicalis* | 0.019 | + | - | - | 13 |
| | | | | | |
| *T. glabrata* | 0.009 | + | + | + | 14 |
| | | | | | |
| *C. parapsilosis* | 0.156 | + | - | - | 15 |
| | | | | | |
| (+ indicates cut-off criterion satisfied; - indicates cut-off criterion not satisfied; cut-off criterion is at least 100-fold reduction in colony count) | | | | | |

The results with *Aspergillus spp* show that there was synergy between Amphotericin B and Mycograb *in vitro* against the commonest *Aspergillus spp.*

### (2) Animal experiments:

Mice infected with the outbreak strain of *Candida albicans* were treated with amphotericin B only (Group 1), amphotericin B and 500 µg anti-hsp90 antibody (Group 2) and amphotericin B and 50 µg anti-hsp90 antibody (Group 3). Yeast colony counts for various tissues from the mice after a treatment period of 48 hours are shown in Table 19. The results show that animals treated with amphotericin B and 500 µg antibody (Group 2) showed a significant reduction (at least one order of magnitude) in the number of yeast counts compared with animals treated with amphotericin B only (Group 1). Animals treated with amphotericin B and 50 µg antibody (Group 3) also showed diminished yeast counts compared with the animals treated with amphotericin B only (Group 1). The *in vivo* data therefore corroborates the *in vitro* data and confirms the synergy between the anti-hsp90 antibody and the antifungal agent amphotericin B for the treatment of fungal infections.

| **Table 19. Colony counts of *C. albicans* (outbreak strain) in tissues of treated mice groups** | | | |
|---|---|---|---|
| | Colonies (cfu/ml, in log10 ± standard deviation) | | |
| | Group 1 | Group 2 | Group 3 |
| Kidney | 6.80 ± 0.916 | 4.42 ± 1.28 | 4.35 ± 1.37 |
| | | | |
| Liver | 4.26 ± 1.42 | 3.22 ± 0.028 | 3.83 ± 1.00 |
| | | | |
| Spleen | 4.18 ± 1.18 | 3.07 ± 0.089 | 3.94 ± 1.25 |

### (3) Clinical Study:

Four patients with evidence of *Candida* infection and who were not responding to conventional antifungal treatment were given a combined treatment of antifungals and anti-hsp90 antibody in the form of Mycograb (see above) and their condition monitored.

Patient 1 was given a primary diagnosis of acute pancreatitis and the patient had postoperative adult respiratory distress syndrome (ARDS) requiring ventilation. *C albicans* was grown *in vitro* from multiple sites including pancreatic bed. The patient had a very high white cell count (WBC) (78.4), although this was highly variable and may not have been caused by the sepsis alone. Abelcet treatment at 3 mg/kg was initiated.

Five days after initiation of Abelcet treatment, Patient 1 was additionally given a first test dose of Mycograb at 0.1 mg/kg (Day 1). On Day 3, the patient was given a clincal dose of Mycograb at 1 mg/kg. Due to several factors, for example a worsening platelet count which had been low for at least four days, the Abelcet and Mycograb treatments were discontinued on Day 3 after the clinical dose of Mycograb had been given. However, Patient 1 regrew *C. albicans* from ascites six days later (Day 9) and was put on fluconazole (400 mg). The following day (Day 10), the patient was given the final two clinical doses of Mycograb at 1 mg/kg per dose.

Although Patient 1 had been on Abelcet for seven days, before the patient received the clinical dose of Mycograb on Day 3, *C*. *albicans* was still being grown from the patient's trachyostomy site and the patient had a tachycardia. The combined treatment with Abelcet and Mycograb on Day 3 resulted in a period of five days during which *C*. *albicans* was not grown. No Mycograb-related changes in blood chemistry, haematology and clotting factors were observed during treatment with Mycograb. Treatment of the subsequent recurrence with fluconazole and Mycograb (two doses on Day 10) was less successful, as would be expected from the *in vitro* synergy results (see for example Tables 2 and 17), but the patient did eventually recover from the candidosis.

Serum levels of Mycograb in Patient 1 at different time intervals following administration of Mycograb doses are shown in Table 20. The test dose at Day 1 did not give measurable serum levels. The 1.0 mg/kg doses at Day 3 and Day 10 did give detectable serum levels, and these levels were comparable with those at which synergy with amphotericin B was demonstrable *in vitro* (see Tables 9 and 18). Following the second dose on Day 10, serum levels of Mycograb improved, indicating some tissue accumulation following the first dose. Mycograb was detectable in the urine at the 1.0 mg/ml doses (data not shown).

| **Table 20. Serum levels (in µg/ml) of Mycograb in Patient 1** | | | | |
|---|---|---|---|---|
| | **Day 1** | **Day 3** | **Day 10** | **Day 10** |
| Time (h) | 0.1 mg/kg | 1.0 mg/kg | 1.0 mg/kg bd | 1.0 mg/kg bd |
| | | | 1^{st} dose | 2^{nd} dose |
| 0 | 0 | 0 | 0 | 0 |
| 0.5 | 0 | 4.0 | 3.0 | 3.0 |
| 1.0 | 0 | 2.5 | 1.2 | 1.4 |
| 2.0 | 0 | 2.5 | 0.5 | 1.0 |
| 4.0 | 0 | 1.0 | 0.3 | 0.4 |
| 6.0 | 0 | - | 0.1 | 0.1 |
| 8.0 | 0 | 0 | 0: 2nd dose then given | 0 |
| 12.0 | 0 | 0 | | |
| 24.0 | 0 | 0 | | |
| 48.0 | 0 | 0 | | |

Patient 2 was diagnosed as having a small bowel constriction due to adhesions and had ARDS requiring ventilation. *C*. *albicans* was grown from multiple sites including ascitic fluid, with the infection associated with a fluctuating temperature (35.8-38.2°C), raised WBC (11.4) and occasional tachycardia (110). The patient was started on Abelcet at 3 mg/kg.

Four days after the commencement of Abelcet treatment, Patient 2 still retained a fluctuating temperature, raised WBC and occasional tachycardia. The patient was given a 0.1 mg/kg test dose of Mycograb (Day 1). The following day, the patient was given a clinical dose of 1 mg/kg Mycograb (Day 2). The last dose of Abelcet was also given on Day 2 due to completion of a 5 day treatment program. Two days later (Day 4), the patient received the final two clinical doses of Mycograb.

The Mycograb was well tolerated by the patient. The clinical dose of Mycograb on Day 2 was associated with a falling and stabilising temperature (38.2 to 36.7°C on Day 2 after receiving the clinical dose, staying at 36.7-37.4°C through to Day 3) and a falling WBC (from 11.9 to 9.6). On Day 4, the patient was looking clinically better and no *C. albicans* was grown from ascites, blood cultures or urine. No Mycograb-related changes in blood chemistry, haematology and clotting factors were observed during treatment. Subsequent recovery was complicated by an episode of bacterial sepsis but this responded to antibiotics and the patient made a full recovery.

Serum levels of Mycograb in Patient 2 at different time intervals following administration of Mycograb doses are shown in Table 21. The test dose at Day 1 did not give measurable serum levels. The 1.0 mg/kg dose at Day 2 did give detectable serum levels, and these levels were compatible with those at which synergy with amphotericin B was demonstrable *in vitro* (see Tables 9 and 18). Mycograb was detectable in the urine at the 1.0 mg/kg doses (data not shown).

| **Table 21. Serum levels (in µg/ml) of Mycograb in Patient 2** | | | | |
|---|---|---|---|---|
| | **Day 1** | **Day 2** | **Day 4** | **Day 4** |
| Time (h) | 0.1 mg/kg | 1.0 mg/kg | 1.0 mg/kg bd | 1.0 mg/kg bd |
| | | | 1^{st} dose | 2^{nd} dose |
| 0 | 0 | 0 | 0 | 0 |
| 0.5 | 0 | 1.5 | 1.0 | 1.0 |
| 1.0 | 0 | 0.5 | 0.5 | 0.5 |
| 2.0 | 0 | 0.3 | 0.1 | 0.5 |
| 4.0 | 0 | 0.1 | 0 | 0.1 |
| 6.0 | 0 | 0 | 0 | 0 |
| 8.0 | 0 | 0 | 0: 2nd dose then given | 0 |
| 12.0 | 0 | 0 | | 0 |
| 24.0 | 0 | 0 | | 0 |
| 48.0 | 0 | 0 | | 0 |

Patient 3 had a six week history of pancreatitis which led to an 80% pancreatectomy. The patient had moderately raised LFT (liver function test) levels, possibly related to alcohol abuse, and was an MRSA (Methicillin-resistant *Staphylococcus aureus*) carrier. *C*. *albicans* were grown from multiple sites so the patient was treated with intravenous fluconazole. Twelve days later, after failing to respond to fluconazole, the patient was changed to 300 mg Abelcet. Three days later, the patient was still pyrexial (38.5°C) and *C*. *albicans* was still growing from multiple sites (abdominal drains and gastroscopy tube), and was therefore given a clinical dose (1 mg/kg) of Mycograb (Day 1) in addition to the Abelcet.

On the same day as the Mycograb dose was given, Patient 3 suffered an acute episode of Gram-negative-type septic shock (high temperature of 39.5°C, hypotensive), probably due to *Pseudomonas aeruginosa,* which was subsequently grown from his pancreatic drain, although he also grew *Enterococcus faecalis* from blood cultures. Due to this episode, no further Mycograb doses were given. The patient subsequently responded to antibiotic therapy (vancomycin and ceftazidime).

Due to the bacterial complications, it was difficult to assess the impact of the single dose of Mycograb on Patient 3. However, it was noted that he stopped growing *C*. *albicans* (for example, from his gastrostomy tube and wound drain) for 48 hours after the dose and that he became apyrexial on Day 2 and Day 3. No Mycograb-related changes in laboratory parameters (blood chemistry, haematology and clotting factors) were observed. On Day 4, the patient had a recurrence of *C*. *albicans* while he was still on Abelcet but a full recovery was made subsequently.

Serum levels of Mycograb in Patient 3 at different time intervals following administration of the Mycograb dose are shown in Table 22. The single 1.0 mg/kg dose at Day 1 gave detectable serum levels, and these levels were compatible with those at which synergy with amphotericin B was demonstrable *in vitro* (see Tables 9 and 18). Mycograb was also detectable in the urine following the 1.0 mg/kg dose (data not shown).

| **Table 22. Serum levels (in µg/ml) of Mycograb in Patient 3** | |
|---|---|
| | **Day 1** |
| Time (h) | 1.0 mg/kg bd |
| 0 | 0 |
| 0.5 | 2.5 |
| 1.0 | 1.5 |
| 2.0 | 1.2 |
| 4.0 | 0.1 |
| 6.0 | 0 |
| 8.0 | 0 |
| 12.0 | 0 |
| 24.0 | 0 |
| 48.0 | 0 |

Patient 4 was diagnosed with *C*. *albicans* empyema, although the patient was originally admitted to ITU (Intensive Treatment Unit) with a lung abscess due to *Streptococcus milleri* (isolated from blood cultures). *C. albicans* was grown from two bronchial lavage specimens (right and left lung) and three and four days later from two empyema fluid specimens. Treatment was started the following day with Abelcet (5 mg/kg). Five days after commencement of Abelcet treatment, some clinical deterioration was noted and the following morning (Day 1) this was associated with high WBC (15.7) and *C*. *albicans* regrown from an intercostal drain fluid.

Mycograb (1 mg/kg bd) was given to Patient 4 at 8.30 am and 8.30 pm on Day 1. Apart from a temporary rise in temperature on the night of Day1, the patient improved clinically. *C*. *albicans* was not grown from empyema fluid specimen cultured on Day 3, and the patient became progressively better.

The Mycograb was well tolerated by Patient 4. No Mycograb-related changes in laboratory parameters (blood chemistry, haematology and clotting factors) were observed. Thus the patient was still growing *C*. *albicans* from a chest drain six days after commencing Abelcet treatment and his WBC was high (15.7) just before receiving the first Mycograb dose, but thereafter the patient steadily improved and stopped growing *C*. *albicans.*

Serum levels of Mycograb in Patient 4 at different time intervals following administration of Mycograb doses are shown in Table 23. The 1.0 mg/kg doses given on Day 1 gave detectable serum levels which were compatible with those at which synergy with amphotericin B was demonstrable *in vitro* (see Tables 9 and 18). Following the second dose on Day 1, serum levels of Mycograb improved, indicating some tissue accumulation following the first dose. Mycograb was detectable in the urine at the 1.0 mg/ml doses (data not shown).

| **Table 23. Serum levels (in µg/ml) of Mycograb in Patient 4** | | |
|---|---|---|
| | **Day 1** | |
| Time (h) | 1.0 mg/kg bd | 1.0 mg/kg bd |
| | 1^{st} dose | 2^{nd} dose |
| 0 | 0 | 8.0 |
| 0.5 | 1.2 | 2.5 |
| 1.0 | 1.2 | 1.4 |
| 2.0 | 0.6 | 1.2 |
| 4.0 | 0.1 | 0.6 |
| 6.0 | 0 | 0.3 |
| 8.0 | 0 | 0.1 |
| 12.0 | - 2nd dose given | 0 |
| 24.0 | - | 0 |
| 48.0 | | 0 |

### Conclusions

The data presented here clearly demonstrates that there is a surprising synergism between the anti-*Candida* hsp90 antibody and the antifungal agent amphotericin B which effects enhanced antifungal activity against a wide variety of pathologically important fungal strains. These results allows for the use of novel, highly effective compositions for the treatment of human or animal fungal infections, and also a novel antibody which can be incorporated into the composition. The present invention allows for either lower treatment dosages or more effective treatment at the same dosages, thereby reducing unwanted side-effects.

Clinical implications of the present invention include: (i) the production of a synergistic combination of amphotericin B and anti-hsp90 antibody in the treatment of disseminated yeast infection should become the treatment of choice. This would lead to a reduction in mortality and morbidity for these infections. The preliminary clinical study results provided herewith confirm the efficacy of the present invention in comparison with existing methods of treatment; (ii) amphotericin B is a toxic, particularly nephrotoxic, drug. The synergy provided by the present invention means that a lower dose of amphotericin B could be used while maintaining efficacy and concomitantly reducing toxicity; and (iii) the toxicity sparing effect of the anti-hsp90 antibody would allow the clinical efficacy of higher doses of amphotericin B to be explored and further contribute to an improved clinical outcome.

## Claims

1. The use of a composition comprising an antibody or an antigen binding fragment thereof specific for one or more epitopes of a fungal stress protein and an antifungal agent comprising at least one of the group consisting of a polyene antifungal agent and an echinocandin antifungal agent in a method of manufacture of a medicament for the treatment of fungal infections, wherein the fungus causing said fungal infection is resistant to said antifungal agent *per se.*

2. The use of a composition, according to claim 1, wherein said antibody is specific for a heat shock protein from a member of the *Candida* or *Torulopsis* genera.

3. The use of a composition according to claim 2, wherein said heat shock protein comprises hsp90 from *Candida albicans.*

4. The use of a composition according to any one of the preceding claims, wherein said antibody or an antigen binding fragment thereof is specific for the epitope comprising the sequence of SEQ ID NO: 1.

5. The use of a composition according to claim 4, wherein said antibody comprises the sequence according to SEQ ID NO: 2.

6. The use of a composition according to any one of the preceding claims, wherein said polyene antifungal agent comprises amphotericin B or a derivative of amphotericin B.

7. The use of a composition according to any one of the preceding claims, wherein said echinocandin antifungal agent comprises Anidulafungin (LY303366).

8. The use of a composition according to any one of the preceding claims, wherein said fungal infection is at least one selected from the group comprising Mucormycosis, Blastomycosis, Coccidioidomycosis, or Paracoccidioidomycosis, or said fungal infection is caused by at least one organism selected from the group comprising *Candida, Cryptococcus, Histoplasma, Aspergillus,* or *Torulopsis* organism.

9. The use of a composition according to either one of claims 4 or 5 wherein said antibody br antigen binding fragment is labelled with a detectable label.

10. The use of a composition according to any of claims 4, 5 or 9, wherein said antibody or antigen binding fragment is conjugated with an effector molecule.

## Patentansprüche

1. Verwendung einer Zusammensetzung eines Antikörpers oder eines Fragmentes eines daran anbindenden Antigens, das spezifisch für ein oder mehrere Epitope eines Pilz-Stressproteins ist, sowie ein Wirkstoff gegen Pilze, enthaltend zumindest einen Stoff ausgewählt aus der Gruppe bestehend aus einem Polyen-Antipilzwirkstoff und einem Echinocandin-Antipilzwirkstoff zur Herstellung eines Medikaments für die Behandlung von Pilzinfektionen, wobei der Pilz, der besagte Pilzinfektion verursacht, *per se* resistent gegen besagten Antipilzwirkstoff ist.

2. Verwendung einer Zusammensetzung nach Anspruch 1, wobei der besagte Antikörper spezifisch für ein Hitzeschockprotein eines Vertreters der Gattungen *Candida* oder *Torulopsis* ist.

3. Verwendung einer Zusammensetzung nach Anspruch 2, wobei besagtes Hitzeschockprotein bsp90 von *Candida albicans* umfasst.

4. Verwendung einer Zusammensetzung nach einem der vorhergehenden Ansprüche, wobei der besagte Antikörper oder das daran anbindende Antigen-Fragment spezifisch für das Epitop, umfassend die Sequenz von SEQ ID NO:1, ist.

5. Verwendung einer Zusammensetzung nach Anspruch 4, wobei der besagte Antikörper die Sequenz gemäß SEQ ID NO:2 umfasst.

6. Verwendung einer Zusammensetzung nach einem der vorhergehenden Ansprüche, wobei der besagte Polyen-Antipilzwirkstoff Amphotericin B oder ein Derivat von Amphotericin B umfasst.

7. Verwendung einer Zusammensetzung nach einem der vorhergehenden Ansprüche, wobei der besagte Echinocandin-Antipilzwirkstoff Anidulafungin (LY303366) umfasst.

8. Verwendung einer Zusammensetzung nach einem der vorhergehenden Ansprüche, wobei die besagte Pilzinfektion mindestens eine, ausgewählt aus der Gruppe bestehend aus *Mucormycosis, Blastomycosis, Coccidioidomycosis* oder *Paracoccidioidomycosis* ist, oder besagte Pilzinfektion durch zumindest einen Organismus, ausgewählt aus der Gruppe bestehend aus *Candida, Cryptococcus, Histoplasma, Aspergillus* oder *Torulopsis* verursacht wird.

9. Verwendung einer Zusammensetzung nach einem der Ansprüche 4 oder 5, wobei der besagte Antikörper oder das daran anbindende Antigen-Fragment mit einer detektierbaren Markierung versehen ist.

10. Verwendung nach einem der Ansprüche 4, 5 oder 9, wobei das besagte Antigen oder das daran anbindende Antigen-Fragment mit einem Effektormolekül konjugiert ist.

## Revendications

1. Utilisation d'une composition comprenant un anticorps ou un fragment de liaison d'antigène de celui-ci spécifique d'un ou plusieurs épitopes d'une protéine de stress fongique et un agent antifongique comprenant au moins l'un du groupe consistant en un agent antifongique polyène et un agent antifongique échinocandine dans un procédé de fabrication d'un médicament pour le traitement des infections fongiques, où le champignon causant ladite infection fongique est résistant audit agent antifongique en soi.

2. Utilisation d'une composition selon la revendication 1, où ledit anticorps est spécifique d'une protéine de choc thermique provenant d'un membre des genres *Candida* ou *Torulopsis*.

3. Utilisation d'une composition selon la revendication 2, où ladite protéine de choc thermique comprend hsp90 provenant de *Candida albicans*.

4. Utilisation d'une composition selon l'une quelconque des revendications précédentes, où ledit anticorps ou un fragment de liaison d'antigène de celui-ci est spécifique de l'épitope comprenant la séquence de SEQ ID NO : 1.

5. Utilisation d'une composition selon la revendication 4, où ledit anticorps comprend la séquence selon SEQ ID NO : 2.

6. Utilisation d'une composition selon l'une quelconque des revendications précédentes, où ledit agent antifongique polyène comprend de l'amphotéricine B ou un dérivé de l'amphotéricine B.

7. Utilisation d'une composition selon l'une quelconque des revendications précédentes, où ledit agent antifongique échinocandine comprend de l'anidulafongine (LY303366).

8. Utilisation d'une composition selon l'une quelconque des revendications précédentes, où ladite infection fongique est au moins une choisie dans le groupe comprenant la mucormycose, la blastomycose, la coccidioidomycose ou la paracoccidioidomycose, ou ladite infection fongique est causée par au moins un organisme choisi dans le groupe comprenant un organisme *Candida, Cryptococcus, Histoplasma, Aspergillus* ou *Torulopsis.*

9. Utilisation d'une composition selon l'une quelconque des revendications 4 et 5, où ledit anticorps ou fragment de liaison d'antigène est marqué avec un marqueur détectable.

10. Utilisation d'une composition selon l'une quelconque des revendications 4, 5 et 9, où ledit anticorps ou fragment de liaison d'antigène est conjugué avec une molécule effectrice.
